# EUROPÄISCHE PATENTANMELDUNG

(11) **EP 2 177 155 A1**
(43) Veröffentlichungstag der Anmeldung: **21.04.2010**
(21) Anmeldenummer: 08167032.5
(22) Anmeldetag: 20.10.2008
(51) Int. Cl.: A61B 5/00, G01N 33/487

(54) **Analytisches Testbandinstrument mit Gleichstrommotor und Getriebe**

(71) Anmelder: F. Hoffmann-La Roche AG, 4070 Basel (CH); Roche Diagnostics GmbH, 68305 Mannheim (DE)
(72) Erfinder: Schosnig, Stefan, 69493 Hirschberg-Grosssachsen (DE); Heck, Wolfgang, 68526 Ladenburg (DE); Porsch, Ulrich, 69469 Weinheim (DE); Lorenz, Robert, 67547 Worms (DE); Kehr, Ulrich, 71116 Gärtringen (DE); Treinzen, Andree, 71296 Heimsheim (DE); Barthold, Ingmar, 73084 Salach (DE)
(74) Vertreter: Pfiz, Thomas

(57) **Zusammenfassung**

Die Erfindung betrifft ein analytisches Testbandinstrument mit einer wechselbaren Testbandeinheit (14), die ein mit einer Vielzahl von mit Körperflüssigkeit beaufschlagbaren Testelementen (18) versehenes Testband (16) umfasst, und einem mit der Testbandeinheit (14) koppelbaren Bandantrieb (12) zum Vorspulen des Testbands (16), so dass die Testelemente (18) sukzessive an einer Applikationsstelle bereitstellbar sind. Erfindungsgemäß wird vorgeschlagen, dass der Bandantrieb (12) einen Gleichstrommotor (26) und ein zwischen dem Gleichstrommotor (26) und der Testbandeinheit (14) angeordnetes Untersetzungsgetriebe (28) aufweist

## Beschreibung

Die Erfindung betrifft ein analytisches Testbandinstrument, insbesondere als Handgerät für Blutzuckertests, mit einer vorzugsweise als Bandkassette ausgebildeten, wechselbaren Testbandeinheit, die ein mit einer Vielzahl von mit Körperflüssigkeit beaufschlagbaren Testelementen versehenes Testband umfasst, und einem mit der Testbandeinheit koppelbaren Bandantrieb zum Vorspulen des Testbands, so dass die Testelemente sukzessive an einer Applikationsstelle bereitstellbar sind.

Derartige Testbandsysteme wurden bereits in einer Reihe von Patentanmeldungen von den Anmeldern vorgeschlagen, um gegenüber den am Markt befindlichen Streifensystemen weitere Anwendervorteile zu gewinnen. Für die Praxis muss neben einer zuverlässigen Positionierung der Testelemente auch gewährleistet sein, dass keine übermäßige Geräuschentwicklung den Einsatz vor Ort beeinträchtigt.

Ausgehend hiervon liegt der Erfindung die Aufgabe zugrunde, die im Stand der Technik vorgeschlagenen Systeme weiter zu verbessern und eine sichere Testelementpositionierung mit geringen Störgeräuschen bei kompaktem Aufbau zu erreichen.

Zur Lösung dieser Aufgabe wird die im Patentanspruch 1 angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, einen kompakten hochdrehenden Motor einzusetzen. Dementsprechend wird erfindungsgemäß vorgeschlagen, dass der Bandantrieb einen Gleichstrommotor und ein zwischen dem Gleichstrommotor und der Testbandeinheit angeordnetes Untersetzungsgetriebe aufweist. Der Gleichstrommotor lässt sich aufgrund seines geringen Bauraums und Energiebedarfs gut in ein Handgerät integrieren und ermöglicht einen geräusch- und schwingungsarmen Betrieb, während das Untersetzungsgetriebe für eine geeignete Drehzahl/Drehmomentwandlung zur zuverlässigen Bandpositionierung sorgt. Als weiterer Vorteil ergibt sich eine vereinfachte Herstellbarkeit bei kostengünstiger Bauweise.

Vorteilhafterweise ist der Gleichstrommotor als mechanisch kommutierter Flachläufer ausgebildet, um ein gleichmäßiges Geräuschverhalten bei geringer Momentenschwankung zu erzielen. Auch lässt sich ein solcher Motor mit wenigen Teilen einfach aufbauen, wobei eine besondere Eignung für den Batteriebetrieb besteht. Denkbar wäre es auch, einen elektronisch kommutierten Gleichstrommotor einzusetzen.

Eine weitere Verbesserung hinsichtlich störender Geräusche und Verschmutzung lässt sich dadurch erreichen, dass das Untersetzungsgetriebe vorzugsweise zusammen mit dem Gleichstrommotor in einem Getriebegehäuse gegenüber der Umgebung gekapselt ist.

Ein besonders kompakter und präziser Aufbau wird vorteilhafterweise dadurch erreicht, dass der Gleichstrommotor ein bereichsweise durch eine Gehäusewand des Getriebegehäuses gebildetes Motorgehäuse aufweist.

Eine weitere vorteilhafte Ausführung sieht vor, dass der Rotor des Gleichstrommotors eine in dem Getriebegehäuse angeordnete Lagerstelle aufweist. Dadurch lassen sich Toleranzketten in Verbindung mit dem nachgeordneten Getriebe weiter verkürzen. Eine weitere Verbesserung in dieser Hinsicht ergibt sich dadurch, dass der Gleichstrommotor eine in einem Motorgehäuseteil angeordnete Lagerstelle für seinen Rotor aufweist, und dass das Motorgehäuseteil in das Getriebegehäuse eingeklemmt ist. Günstig ist es auch, wenn das mit dem Gleichstrommotor direkt gekoppelte Getriebeglied des Untersetzungsgetriebes in einer Gehäusepartie des Gleichstrommotors gelagert ist.

Zur Vermeidung von Körperschallbrücken ist es von Vorteil, wenn die Testbandeinheit auf ein Gerätechassis aufsetzbar ist, wobei das Getriebegehäuse nur punktuell unter Freihaltung eines Luftspalts mit dem Gerätechassis verbunden ist.

Um die Antriebskräfte zuverlässig übertragen zu können, ist es von Vorteil, wenn Untersetzungsgetriebe als Zahnradgetriebe, insbesondere als mehrstufiges Stirnradgetriebe mit z.B. seitlich versetztem An- und Abtrieb ausgebildet ist. Hierbei ist es auch für eine kostengünstige Herstellung günstig, wenn die Zahnräder des Zahnradgetriebes als Spritzgussteile aus Kunststoff, insbesondere POM bestehen.

Um das Testband unabhängig vom Wickeldurchmesser möglichst gleichmäßig zu transportieren, ist es vorteilhaft, wenn der Bandantrieb einen Drehzahlregler zur Regelung der Drehzahl des Gleichstrommotors in Abhängigkeit von der Anzahl der bereitgestellten Testelemente der Testbandeinheit aufweist. Eine weitere vorteilhafte Ausgestaltung sieht vor, dass die Motordrehzahl des Gleichstrommotors im Bereich zwischen 100 und 200 Umdrehungen/s liegt, und dass die Ausgangsdrehzahl des Untersetzungsgetriebes zwischen 0,2 und 0,5 Umdrehungen/s beträgt

Vorteilhafterweise ist der Gleichstrommotor über Bürsten an eine batteriegespeiste Energieversorgung angeschlossen, wobei Bürstenhalterungen im Getriebegehäuse zur Dämpfung von Bürstenschwingungen integriert sind.

Eine weitere Minimierung der Motorschwingungen ist dadurch erreichbar, dass der Gleichstrommotor eine an einem Endabschnitt längsgeschlitzte Motorwelle aufweist, so dass der geschlitzte Endabschnitt unter Toleranzausgleich in eine Lagerbohrung einsetzbar ist.

Von Vorteil hinsichtlich reduzierter Lagerreibung und winkeltreuer Bewegungsübertragung ist es auch, wenn die Motorwelle des Gleichstrommotors vorzugsweise einstückig mit einem Antriebsritzel aus Kunststoff, insbesondere POM ausgebildet ist.

Ein wesentlicher Vorteil des erfindungsgemäßen Bandantriebs liegt neben der Geräuschminimierung auch in der Realisierung einer kompakten Bauweise. Demgemäß ist es möglich, dass ein den Bandantrieb umhüllendes und die Testbandeinheit aufnehmendes Gehäuse einen Bauraum von weniger als 150 cm³, vorzugsweise etwa 135 cm³ umschließt.

Im Folgenden wird die Erfindung anhand eines in der Zeichnung dargestellten Ausführungsbeispiels näher erläutert. Es zeigen:
- Fig. 1: ein analytisches Testbandinstrument mit wechselbarer Bandkassette in einer perspektivischen Darstellung;
- Fig. 2: den Bandantrieb des Testbandinstruments in ausschnittsweiser Vergrößerung der Fig. 1;
- Fig. 3: den Bandantrieb im Schnitt entlang einer durch die Zahnrad-Drehachsen verlaufenden Schnittlinie;
- Fig. 4: den Motor des Bandantriebs im Axialschnitt;
- Fig. 5: die endseitig geschlitzte Motorwelle in einer perspektivischen Ausschnittsvergrößerung der Fig. 4.

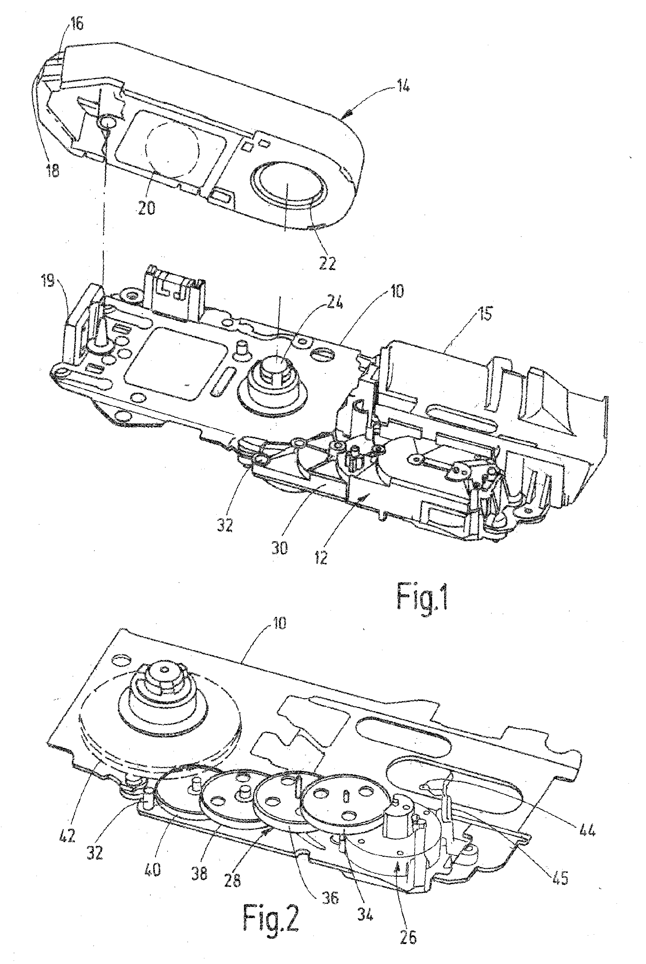

Das in Fig. 1 dargestellte Testbandinstrument umfasst eine Geräteeinheit 10 mit einem besonders laufruhig ausgestalteten Bandantrieb 12 und eine wechselbare Bandkassette 14, die mit dem Bandantrieb koppelbar ist. Von der Geräteinheit 10 ist der Übersichtlichkeit halber das Chassis mit nur ausschnittsweise umhüllendem Gehäuse 15 gezeigt. Das Testbandinstrument lässt sich als Handgerät für Blutzuckermessungen einsetzen, die vor Ort vom Anwender selbst vorgenommen werden können. Diesbezügliche Einzelheiten ergeben sich beispielsweise aus der EP-A 1 760 469, auf die in diesem Zusammenhang Bezug genommen wird.

Die in Fig. 1 in gedrehter Perspektive von unten gezeigte Bandkassette 14 enthält ein Testband 16, das abschnittsweise mit Testfeldern 18 versehen ist, die im Bereich einer Umlenkspitze als Applikationsstelle an ihrer freien Vorderseite mit Blut bzw. Körperflüssigkeit beaufschlagbar sind. Zugleich kann dort eine rückseitige Vermessung des jeweiligen Testfelds 18 durch die Messeinheit 19 erfolgen. Das Testband 16 wird zu diesem Zweck mittels des Bandantriebs 12 von einer Abwickelspule 20 auf eine Aufwickelspule 22 umgespult, so dass die voneinander beabstandeten Testfelder 18 nacheinander an der Applikationsstelle für sukzessive Tests zum Einsatz gebracht werden. Hierbei wird nur die Aufwickelspule 22 durch den formschlüssig eingreifenden Antriebszapfen 24 angetrieben.

Wie auch aus Fig. 2 ersichtlich, weist der Bandantrieb 12 einen als Gleichstrommotor 26 ausgebildeten Elektromotor und ein nachgeordnetes Untersetzungsgetriebe 28 auf. Das Untersetzungsgetriebe 28 ist zusammen mit dem Motor 26 in einem Getriebegehäuse 30 gegenüber der Umgebung gekapselt bzw. abgeschirmt. Um eine Körperschallübertragung zu unterdrücken, ist das Getriebegehäuse 30 nur an einzelnen Befestigungspunkten 32 mit dem darunter liegenden Chassis 10 verbunden, wobei ein Luftspalt freigehalten wird.

Vorteilhafterweise ist das Getriebegehäuse aus einem LCP (Liquid Crystal Polymer) ausgebildet, also einem hochschmelzenden Werkstoff, der enge Toleranzen erlaubt. Ggf. bestehen auch andere Aufbauten an dem Chassis 10 aus diesem Material, um einen übereinstimmenden Längenausdehnungskoeffizienten sicherzustellen.

Das Untersetzungsgetriebe 28 besteht aus mehreren Zahnrädern 34, 36, 38, 40 mit zueinander parallelen Drehachsen, die somit als mehrstufiges Stirnradgetriebe einen seitlich versetzten An- und Abtrieb aufweisen. Ein weiteres Zahnrad 42 an der Unterseite des Chassis 10 ist koaxial mit dem Antriebszapfen 24 verbunden. Die Zahnräder bestehen zweckmäßig als Spritzgussteile aus POM (Polyoxymethylen). Im Sinne einer weiteren Geräuschreduzierung ist es vorteilhaft, wenn die Zahnfußtiefe des im Eingriff mit dem Rotor befindlichen ersten Zahnrads 34 erhöht ist, so dass die Zähne etwas länger ausgestaltet sind. Dadurch sind die Zähne weniger steif und dämpfen somit Schwingungen.

Um eine ungewollte Schallübertragung weiter einzuschränken, wird der Motor 26 zweckmäßig über schwingungsgedämpfte Bürsten 44 mit Energie versorgt. Zu diesem Zweck sind im Getriebegehäuse 30 hinreichend steife Bürstenhalterungen 45 integriert, um hohe Schwingungsfrequenzen zu vermeiden. Zusätzlich können auch Teile der Bürsten 44 im Bereich des Unterkastens 48 mit Fett versehen werden, um insbesondere hochfrequente Schwingungen zu dämpfen.

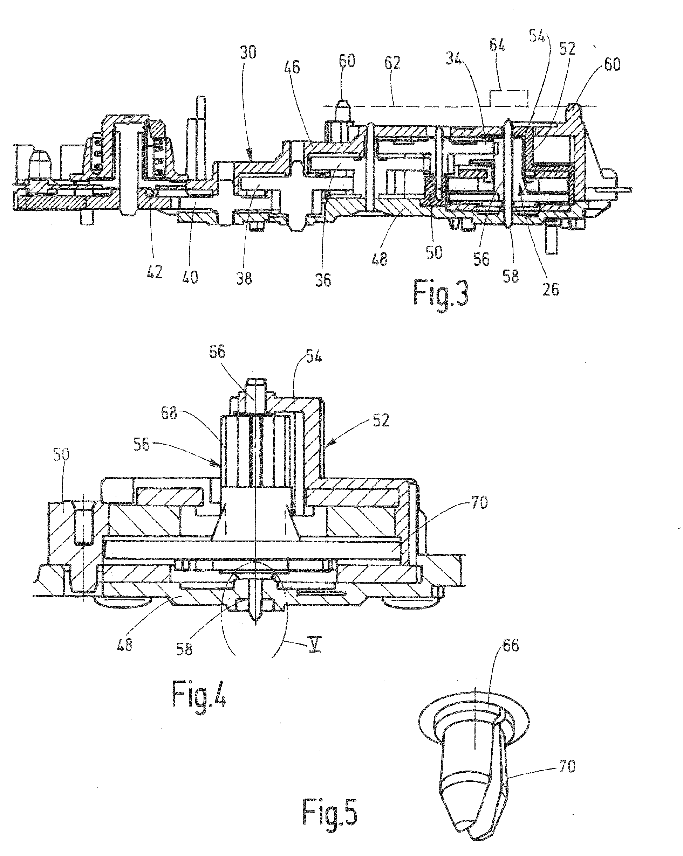

Wie aus Fig. 3 ersichtlich, weist das Getriebegehäuse 30 einen Oberkasten 46 und einen Unterkasten 48 auf, zwischen denen die Zahnräder 36, 38 und 40 gelagert sind. Hingegen ist das mit dem Motor 26 direkt gekoppelte erste Zahnrad 34 an seiner nach unten abstehenden Achse in einer Gehäusepartie 50 des Motorgehäuses 52 gelagert, so dass dort Positioniertoleranzen weitgehend minimiert sind. Ein weiterer domartiger Motorgehäuseteil 54, welcher die obere Lagerstelle für den Motor-Rotor 56 bildet, ist in einer Öffnung des Oberkastens 46 fest eingeklemmt, um auch dort eine genaue Zentrierung und eine Stabilisierung sicherzustellen.

Das Motorgehäuse 52 ist nicht allseitig geschlossen, sondern stirnseitig nach unten offen, um eine möglichst kompakte Anordnung zu schaffen. Die Öffnung wird dabei durch den Unterkasten 48 abdeckt, der somit das Motorgehäuse ergänzt und eine Lagerstelle 58 für den Rotor 56 bildet.

Der Oberkasten 46 ist über Punktverbindungen 60 mit einer nur symbolisch dargestellten Steuerplatine 62 verbunden, die einen Drehzahlregler 64 für den Motor 26 aufweist. Damit lässt sich die Motordrehzahl nach Maßgabe des Wickeldurchmesser entsprechend der Anzahl verbrauchter Tests so steuern, dass eine gleich bleibende Bandtransportgeschwindigkeit von ca. 15 mm/s erreicht wird. Die Motordrehzahlen liegen dabei im Bereich von ca. 100 bis 200 pro Sekunde, wobei durch die Untersetzung auf eine Ausgangsdrehzahl des Antriebszapfens 24 von ca. 0,2 bis 0,4 Umdrehungen in der Sekunde auch das erforderliche Drehmoment zur Verfügung steht.

Wie am besten aus Fig. 4 ersichtlich, ist die Motorwelle 66 mit einem Ritzel 68, das mit dem ersten Zahnrad 34 kämmt, einstückig aus Kunststoff, insbesondere POM ausgebildet. Durch den eisenlosen Rotor wird neben einer hohen Dynamik ein rastmomentfreier Lauf erreicht. Für den Rotor kommt ein mechanisch kommutierter Flachläufer 70 zum Einsatz, der neben einer geringen Baulänge eine niedrige Anlaufspannung bei geringem Energieverbrauch besitzt, was in einem tragbaren Gerät für den Batteriebetrieb von besonderem Vorteil ist.

Die untere Lagerstelle 58 des Rotors 56 ist durch eine Bohrung in dem Unterkasten 48 gebildet. Wie in der Detailansicht nach Fig. 5 gezeigt, kann die Motorwelle 66 einen längsgeschlitzten Endabschnitt 70 aufweisen, der durch radiale Spreizung unter Toleranzausgleich in die Lagerbohrung einsetzbar ist, um eine besondere Laufruhe zu gewährleisten. Hierbei ist zu bedenken, dass in diesem Bereich Vibrationen der Motorwelle zu einer hohen Geräuschentwicklung führen, während im oberen Wellenabschnitt durch die Zahnradkräfte eine Vorzugsrichtung in der Lagerstelle resultiert.

Vor allem das erste hochdrehende Zahnrad 34 erfährt durch den Zahneingriff eine Radialkraft in einer Vorzugsrichtung. Um hierbei die Lagerbedingungen zu verbessern, kann anstelle einer zylindrischen Lagerbohrung ein Lagerauge mit einer Prismenführung für die Zahnradwelle vorgesehen sein.

Für denn Benutzer ist es von großem Vorteil, dass durch den kompakten Bandantrieb 12 auch ein sehr kleines Gerätegehäuse 15 realisierbar ist. Zweckmäßig umfasst das umschlossenen Gerätevolumen etwa 135 cm³, während eine zusätzlich angekoppelte Stechhilfe (optional; nicht gezeigt) ein Bauvolumen von ca. 20 cm³ aufweist, so dass ein Gesamtvolumen von ca. 155 bis 160 cm³ resultiert.

## Patentansprüche

1. Analytisches Testbandinstrument, insbesondere für Blutzuckertests, mit einer vorzugsweise als Bandkassette ausgebildeten, wechselbaren Testbandeinheit (14), die ein mit einer Vielzahl von mit Körperflüssigkeit beaufschlagbaren Testelementen (18) versehenes Testband (16) umfasst, und einem mit der Testbandeinheit (14) koppelbaren Bandantrieb (12) zum Vorspulen des Testbands (16), so dass die Testelemente (18) sukzessive an einer Applikationsstelle bereitstellbar sind, **dadurch gekennzeichnet, dass** der Bandantrieb (12) einen Gleichstrommotor (26) und ein zwischen dem Gleichstrommotor (26) und der Testbandeinheit (14) angeordnetes Untersetzungsgetriebe (28) aufweist.

2. Testbandinstrument nach Anspruch 1, **dadurch gekennzeichnet, dass** der Gleichstrommotor (26) als mechanisch kommutierter Flachläufer ausgebildet ist.

3. Testbandinstrument nach Anspruch 1 oder 2, **dadurch gekennzeichnet, dass** das Untersetzungsgetriebe (28) vorzugsweise zusammen mit dem Gleichstrommotor (26) in einem Getriebegehäuse (30) gegenüber der Umgebung gekapselt ist.

4. Testbandinstrument nach einem der Ansprüche 1 bis 3, **dadurch gekennzeichnet, dass** der Gleichstrommotor (26) ein bereichsweise durch eine Gehäusewand (48) des Getriebegehäuses (30) gebildetes Motorgehäuse (52) aufweist.

5. Testbandinstrument nach einem der Ansprüche 1 bis 4, **dadurch gekennzeichnet, dass** der Rotor (56) des Gleichstrommotors (26) eine in dem Getriebegehäuse (30) angeordnete Lagerstelle (58) aufweist.

6. Testbandinstrument nach einem der Ansprüche 1 bis 5, **dadurch gekennzeichnet, dass** der Gleichstrommotor (26) eine in einem Motorgehäuseteil (54) angeordnete Lagerstelle für seinen Rotor (56) aufweist, und dass das Motorgehäuseteil (54) in das Getriebegehäuse (30) eingeklemmt ist.

7. Testbandinstrument nach einem der Ansprüche 1 bis 6, **dadurch gekennzeichnet, dass** das mit dem Gleichstrommotor (26) direkt gekoppelte Getriebeglied (34) des Untersetzungsgetriebes (28) in einer Gehäusepartie (50) des Gleichstrommotors (26) gelagert ist.

8. Testbandinstrument nach einem der Ansprüche 1 bis 7, **dadurch gekennzeichnet, dass** die Testbandeinheit (14) auf ein Gerätechassis (10) aufsetzbar ist, und dass das Getriebegehäuse (30) nur punktuell unter Freihaltung eines Luftspalts mit dem Gerätechassis (10) verbunden ist.

9. Testbandinstrument nach einem der Ansprüche 1 bis 8, **dadurch gekennzeichnet, dass** das Untersetzungsgetriebe (28) als Zahnradgetriebe, insbesondere als mehrstufiges Stirnradgetriebe mit seitlich versetztem An- und Abtrieb ausgebildet ist.

10. Testbandinstrument nach Anspruch 9, **dadurch gekennzeichnet, dass** die Zahnräder (34,36,38,40) des Zahnradgetriebes (28) als Spritzgussteile aus Kunststoff, insbesondere POM bestehen.

11. Testbandinstrument nach einem der Ansprüche 1 bis 10, **dadurch gekennzeichnet, dass** der Bandantrieb (12) einen Drehzahlregler (64) zur Regelung der Drehzahl des Gleichstrommotors (26) in Abhängigkeit von der Anzahl der bereitgestellten Testelemente der Testbandeinheit (14) aufweist.

12. Testbandinstrument nach einem der Ansprüche 1 bis 11, **dadurch gekennzeichnet, dass** die Motordrehzahl des Gleichstrommotors (26) im Bereich zwischen 100 und 200 Umdrehungen/s liegt, und dass die Ausgangsdrehzahl des Untersetzungsgetriebes (28) zwischen 0,2 und 0,5 Umdrehungen/s beträgt.

13. Testbandinstrument nach einem der Ansprüche 1 bis 12, **dadurch gekennzeichnet, dass** der Gleichstrommotor (26) über Bürsten (44) an eine batteriegespeiste Energieversorgung angeschlossen ist, und dass Bürstenhalterungen (45) im Getriebegehäuse (30) zur Dämpfung von Bürstenschwingungen integriert sind.

14. Testbandinstrument nach einem der Ansprüche 1 bis 13, **dadurch gekennzeichnet, dass** der Gleichstrommotor (26) eine an einem Endabschnitt (70) längsgeschlitzte Motorwelle (66) aufweist, so dass der geschlitzte Endabschnitt (70) unter Toleranzausgleich in eine Lagerbohrung einsetzbar ist.

15. Testbandinstrument nach einem der Ansprüche 1 bis 14, **dadurch gekennzeichnet, dass** die Motorwelle (66) des Gleichstrommotors (26) vorzugsweise einstückig mit einem Antriebsritzel (68) aus Kunststoff, insbesondere POM ausgebildet ist.

16. Testbandinstrument nach einem der Ansprüche 1 bis 15, **dadurch gekennzeichnet, dass** ein umhüllendes Gehäuse (15) einen Bauraum von weniger als 150 cm³, vorzugsweise etwa 135 cm³ umschließt.
